# EUROPEAN PATENT APPLICATION

(11) **EP 2 474 816 A1**
(43) Date of publication of application: **11.07.2012**
(21) Application number: 10290363.0
(22) Date of filing: 30.06.2010
(51) Int. Cl.: G01F 1/36, G01F 1/74

(54) **An apparatus for measuring at least one characteristic value of a multiphase fluid mixture**

(71) Applicant: Services Pétroliers Schlumberger, 75007 Paris (FR); Schlumberger Technology B.V., 2514 JG The Hague (NL); Schlumberger Holdings Limited (SHL), Tortola (VG); PRAD Research and Development Limited, Road Town, Tortola (VG)
(72) Inventor: Lupeau, Alexandre, 5862 Bergen (NO); Baker, Andrew, 5226 Nesttun (NO)
(74) Representative: Vandermolen, Mathieu

(57) **Abstract**

An apparatus (1) for measuring at least one characteristic value of a multiphase fluid mixture (13) comprises:
- a pipe section (20) having a main flow path through which the multiphase fluid mixture (13) flows, the main flow path comprising a throat (22) between an upstream part (21) and a downstream part (23) of the main flow path such as to generate a pressure drop between the upstream part (21) and the downstream part (23);
- a first sensing arrangement (28, 33, 29, 34, 35, 36) to measure a first characteristic value of the multiphase fluid mixture (13) flowing into the main flow path;
- a channel (25) being positioned within the wall of the pipe section (20) such as to maintain similar operating conditions as in the main flow path, the channel (25) being coupled to the main flow path by an inlet (24) at the upstream part (21) and by an outlet (26) at the downstream part (23) such that the pressure drop generates an aspiration effect diverting a sample of the multiphase fluid mixture (13) flowing through the main flow path into the channel (25), the channel (25) having an internal diameter (70) such that said multiphase fluid mixture sample flows in successive separated enriched phase samples (42, 52) within the channel (25); and
- a second sensing arrangement (30) to measure a second characteristic value of one of the phase of the multiphase fluid mixture sample flowing into the channel (25).

## Description

### TECHNICAL FIELD

An aspect of the present invention relates to an apparatus for measuring at least one characteristic value of a multiphase fluid mixture, for example an apparatus for measuring a flow rate of a multiphase fluid mixture. Such a measuring apparatus may be used, in particular but not exclusively, in oilfield related applications, for example, to measure a flow rate of a hydrocarbon effluent flowing out of a geological formation into a well that has been drilled for the purpose of hydrocarbon exploration and production.

### BACKGROUND OF THE INVENTION

WO 99/10712 describes a flow rate measurement method adapted to oil effluents made up of multiphase fluid mixtures comprising water, oil, and gas. The effluent is passed through a Venturi in which the effluent is subjected to a pressure drop (Δp), a mean value <Δp> of the pressure drop is determined over a period t₁ corresponding to a frequency f₁ that is low relative to the frequency at which gas and liquid alternate in a slug flow regime, a means value <pm> is determined for the density of the fluid mixture at the constriction of the Venturi over said period t₁, and a total mass flow rate value <Q> is deduced for the period t₁ under consideration from the mean values of pressure drop and of density.

Limitations of the accuracy of such a multiphase flow rate measurement may occur when the amount of one or several phases of the mixture become very low. In particular, this may occur when the Gas Volume Fraction (GVF) in the Venturi section becomes very high, for example up to 95%. Further, in high gas volume fraction situation, the properties of the liquid phase cannot be estimated. However, estimating the properties of the liquid phase is important. As a first example, estimating the water liquid ratio of the liquid phase helps detecting formation water breakthrough in the well bore. As a second example, estimating the water salinity of the liquid phase helps correcting the flow rate measurements for better accuracy.

### SUMMARY OF THE INVENTION

It is an object of the present invention to propose an apparatus for measuring at least one characteristic value of a multiphase fluid mixture that overcomes one or more of the limitations of the existing multiphase fluid mixture measuring apparatus.

According to one aspect of the present invention, there is provided an apparatus for measuring at least one characteristic value of a multiphase fluid mixture comprising:
- a pipe section having a main flow path through which the multiphase fluid mixture flows, the main flow path comprising a throat between an upstream part and a downstream part of the main flow path such as to generate a pressure drop between the upstream part and the downstream part;
- a first sensing arrangement to measure a first characteristic value of the multiphase fluid mixture flowing into the main flow path;
- a channel being positioned within the wall of the pipe section such as to maintain similar operating conditions as in the main flow path, the channel being coupled to the main flow path by an inlet at the upstream part and by an outlet at the downstream part such that the pressure drop generates an aspiration effect diverting a sample of the multiphase fluid mixture flowing through the main flow path in the channel, the channel having an internal diameter such that said multiphase fluid mixture sample flows in successive separated enriched phase samples within the channel;
- a second sensing arrangement to measure a second characteristic value of one of the phase of the multiphase fluid mixture sample flowing into the channel.

The apparatus may further comprise a valve arrangement regulating a flow suction of the multiphase fluid mixture into the channel. The valve arrangement may be positioned within the channel closed to the inlet.

The apparatus may be positioned such that the channel is substantially parallel to the gravity direction.

The first sensing arrangement may comprise pressure tapings and pressure sensors for measuring the differential pressure of the multiphase fluid mixture between the upstream part and the throat and estimating a total flowrate of the multiphase fluid mixture.

The first sensing arrangement may comprise a gamma rays source and a gamma rays detector for measuring an absorption of the gamma rays by each phase of the multiphase fluid mixture and estimating a density of the multiphase fluid mixture and a fractional flowrate for each phase.

The second sensing arrangement may be an optical sensor or an electromagnetic sensor.

The multiphase fluid mixture may be a hydrocarbon effluent comprising gas, oil, and water.

The second characteristic value of a liquid phase is chosen among the group of characteristic values comprising a water salinity, water liquid ratio, formation water identification, injection water identification, completion liquid identification.

An inline pump may further be disposed within the channel so as to sustain the aspiration effect.

According to another aspect of the present invention, there is provided a method for measuring at least one characteristic value of a multiphase fluid mixture comprising:
- generating a pressure drop between an upstream part and a downstream part by letting flow the multiphase fluid mixture in a pipe section having a main flow path comprising a throat positioned between the upstream part and the downstream part of the main flow path;
- measuring a first characteristic value of the multiphase fluid mixture flowing into the main flow path by means of a first sensing arrangement;
- diverting a sample of the multiphase fluid mixture flowing through the main flow path in a channel by means of an aspiration effect generated by the pressure drop, the channel being positioned within the wall of the pipe section such as to maintain similar operating conditions as in the main flow path, the channel being coupled to the main flow path by an inlet at the upstream part and by an outlet at the downstream part;
- dimensioning an internal diameter of the channel such that said multiphase fluid mixture sample flows in successive separated enriched phase samples within the channel; and
- measuring a second characteristic value of one of the phase of the multiphase fluid mixture sample flowing into the channel by means of a second sensing arrangement.

The method may further comprise regulating the flow in the channel by means of a valve arrangement, the regulation comprising adjusting the residence time of the multiphase fluid mixture sample within the channel such as to obtain free gas liquid samples.

The method may further comprise positioning the channel substantially parallel to the gravity direction.

The method may further comprise correcting the measured first characteristic value of the multiphase fluid mixture flowing into the main flow path based on the measured second characteristic value of one of the phase of the multiphase fluid mixture flowing into the channel.

The measurement of the first characteristic value of the multiphase fluid mixture flowing into the main flow path may comprise measuring the differential pressure of the multiphase fluid mixture between the upstream and downstream parts and estimating a total flowrate of the multiphase fluid mixture.

The measurement of the first characteristic value of the multiphase fluid mixture flowing into the main flow path may comprise submitting the multiphase fluid mixture to gamma rays, measuring an absorption of the gamma rays by each phase of the multiphase fluid mixture and estimating a fractional flowrate for each phase.

The measurement of the second characteristic value of the liquid phase may comprise measuring a water salinity, or measuring a water liquid ratio, or identifying a formation water, or identifying an injection water, or identifying a completion liquid.

The multiphase fluid mixture measuring apparatus of the invention enables performing additional measurements related to the multiphase fluid mixture on a representative sample within a time scale that is similar to the flow rate and density measurements related to the multiphase fluid mixture in the main flow path.

Further, it solves the problem of the rarefaction of one of the phases of the multiphase fluid mixture in case of high GVF situation by extracting an enriched liquid sample from the main flow path. The measurements of the properties of the extracted enriched liquid sample are performed at the same operating conditions than the flow rate and density measurements related to the multiphase fluid mixture in the main flow path.

Furthermore, the measuring apparatus is fully non intrusive, the diverted multiphase fluid mixture sample returning back to the main flow path.

Other advantages will become apparent from the hereinafter description of the present invention.

### BRIEF DESCRIPTION OF THE DRAWINGS

The present invention is illustrated by way of examples and not limited to the accompanying drawings, in which like references indicate similar elements:
- Figure 1 schematically shows an onshore hydrocarbon well location illustrating examples of deployment of the measuring apparatus of the invention;
- Figure 2 is a cross-section view schematically illustrating the measuring apparatus of the invention;
- Figure 3 is a cross-section view in a pipe section schematically illustrating a situation of high gas volume fraction GVF;
- Figure 4 is a cross-section view schematically illustrating an upstream part of the measuring apparatus of the invention; and
- Figure 5 is a cross-section view schematically illustrating a downstream part of the measuring apparatus of the invention.

### DETAILED DESCRIPTION OF THE INVENTION

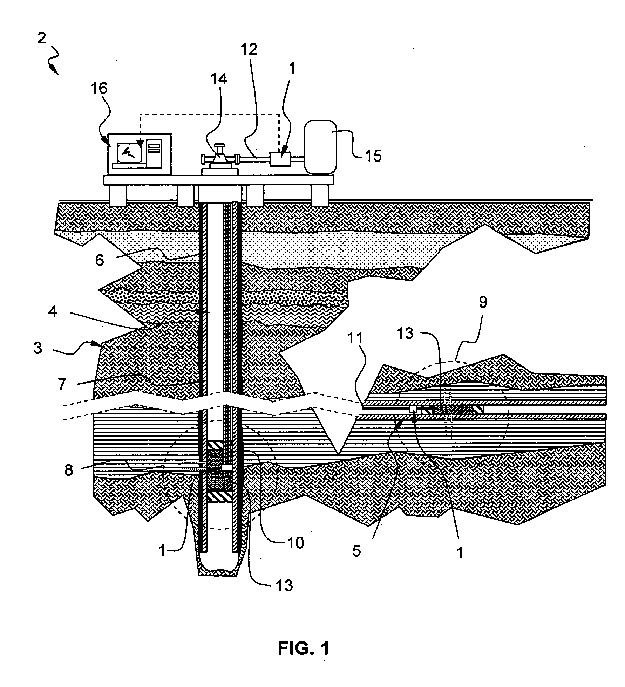

FIG. 1 schematically shows an onshore hydrocarbon well location and equipments 2 above a hydrocarbon geological formation 3 after drilling operation has been carried out, after a drill pipe has been run, and eventually, after cementing, completion and perforation operations have been carried out, and exploitation has begun. The well is beginning producing hydrocarbon, e.g. oil and/or gas. At this stage, the well bore comprises substantially vertical portion 4 and may also comprise horizontal or deviated portion 5. The well bore 4 is either an uncased borehole, or a cased borehole, or a mix of uncased and cased portions.

The cased borehole portion comprises an annulus 6 and a casing 7. The annulus 6 may be filled with cement or an open-hole completion material, for example gravel pack. Downhole, a first 8 and second 9 producing sections of the well typically comprises perforations, production packers and production tubings 10, 11 at a depth corresponding to a reservoir, namely hydrocarbon-bearing zones of the hydrocarbon geological formation 3. A fluid mixture 13 flows out of said zones 8, 9 of the hydrocarbon geological formation 3. The fluid mixture 13 is a multiphase hydrocarbon fluid mixture comprising a plurality of fluid fractions (water, oil, gas) and a plurality of constituting elements (water, various hydrocarbon molecules, various molecules solved in water). The fluid mixture 13 flows downhole through the production tubings 11, 12 and out of the well from a well head 14. The well head 14 is coupled to surface production arrangement 15 by a surface flow line 12 The surface production arrangement 15 may typically comprise a chain of elements connected together, e.g. a pressure reducer, a heat exchanger, a pumping arrangement, a separator, a tank, a burner, etc ... (not shown in details). In one embodiment, one or more apparatus 1 for measuring at least one characteristic value of the multiphase fluid mixture 13 may be installed into the production tubings 10 associated with the first producing section 8, or into the production tubings 11 associated with the second producing section 9 (as represented in FIG. 1) or other sections of the well (not represented in FIG. 1). In another embodiment, one or more apparatus 1 for measuring at least one characteristic value of the multiphase fluid mixture 13 may be installed within the surface flow line 12.

A control and data acquisition arrangement 16 is coupled to the measuring apparatuses 1 of the invention, and/or to other downhole sensors (not shown) and/or to active completion devices like valves (not shown). The control and data acquisition arrangement 16 may be positioned at the surface. The control and data acquisition arrangement 16 may comprise a computer. It may also comprise a satellite link (not shown) to transmit data to a client's office. It may be managed by an operator.

The precise design of the down-hole producing arrangement and surface production/control arrangement is not germane to the present invention, and thus these arrangements are not described in detail herein.

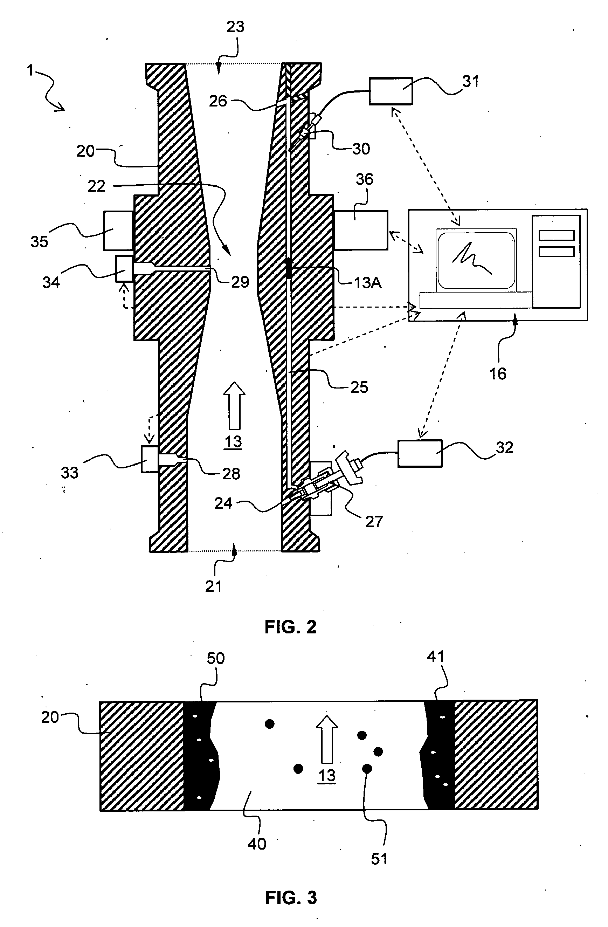

Figure 2 is a cross-section view schematically illustrating an embodiment of the measuring apparatus 1 of the invention. The main purpose of the measuring apparatus is to measure the flow rates of a commingled flow of different phases 13, e.g. gas, oil and water, without separating the phases. The measuring apparatus performs additional measurements, in particular on the liquid phase.

The measuring apparatus 1 comprises a pipe section 20 which internal diameter gradually decreases from an upstream part 21 to a throat 22, forming a convergent Venturi, then gradually increases from the throat 22 to a downstream part 23. The convergent Venturi induces a pressure drop between the upstream part 21 and the downstream part 23 encompassing the throat 22. The pipe section 20 can be coupled to any flowing line 10, 11, 12 by any appropriate connection arrangement (not shown), for example a flange having a bolt-hole pattern and a gasket profile. The upstream part 21, the throat 22 and the downstream part 23 of the pipe section 20 define a main flow path through which the multiphase fluid mixture 13 flows.

Further, the measuring apparatus 1 comprises a channel 25. Advantageously, the channel 25 is positioned within the wall of the pipe section 20. This enables having similar operating conditions (e.g. temperature, pressure) in the channel 25 relatively to the main flow path 21, 22, 23. The channel 25 is coupled to the main flow path by an inlet 24 at the upstream part 21 and by an outlet 26 at the downstream part 23. The channel 25 constitutes a bypass of the throat 22. The pressure drop induced by the convergent Venturi generates an aspiration effect diverting an amount 13A of the multiphase fluid mixture 13 flowing through the main flow path 21, 22, 23 into the channel 25. The amount of the multiphase fluid mixture 13 flows into the channel 25 through the inlet 24 at the upstream part 21 and return back to the main flow path through the outlet 26 at the downstream part 23.

As an alternative (not shown), the aspiration effect may also be further sustained or increased by means of an inline pump, for example disposed within the channel 25.

Furthermore, a flow restriction means may be fitted within the channel 25. The flow restriction may be adjustable: As an example, an adjustable flow restriction under the form of a valve arrangement 27 is depicted on the drawings. The valve arrangement 27 regulates the flow suction of the multiphase fluid mixture 13 into the channel 25. As an example, the valve arrangement 27 is positioned closed to the inlet 24.

Furthermore, the measuring apparatus 1 comprise a first sensing arrangement to measure a first characteristic value of the multiphase fluid mixture 13 flowing into the main flow path 21, 22, 23, and a second sensing arrangement to measure a second characteristic value of one of the phase of the multiphase fluid mixture sample flowing into the channel 25.

In an embodiment, the first sensing arrangement is a Venturi flowmeter estimating a total flowrate of the multiphase fluid mixture based on differential pressure measurement. The pipe section 20 is provided with pressure tappings 28, 29. A first pressure tapping 28 is positioned in the upstream part 21. A first pressure sensor 33 is associated with the first pressure tapping 28 for measuring the pressure of the multiphase fluid mixture 13 flowing in the upstream part 21. A second pressure tapping 29 is positioned at the throat 22. A second pressure sensor 34 is associated with the second pressure tapping 29 for measuring the pressure of the multiphase fluid mixture 13 flowing at the throat 22. Thus, the pressure drop of the multiphase fluid mixture between the upstream part and the throat due to the convergent Venturi can be measured.

In another embodiment, the first sensing arrangement comprises a gamma rays source 35 and a gamma rays detector 36 forming a gamma densitometer. The gamma densitometer measures an absorption of the gamma rays by each phase of the multiphase fluid mixture and estimates a density of the multiphase fluid mixture 13 and a fractional flowrate for each phase. The gamma rays source 35 and the gamma rays detector 36 are diametrically positioned on each opposite sides of the throat 22 or closed to the throat.

The gamma rays source 35 may be a radioisotope Barium 133 source. Such a gamma rays source generates photons which energies are distributed in a spectrum with several peaks. The main peaks have three different energy levels, namely 32 keV, 81 keV and 356 keV. As another example, a known X-Ray tube may be used as a gamma rays source 35.

The gamma rays detector 36 comprises a scintillator crystal (e.g. NaITI) and a photomultiplier. The gamma rays detector 36 measures the count rates (the numbers of photons detected) in the various energy windows corresponding to the attenuated gamma rays having passed through the multiphase fluid mixture 13 at the throat. More precisely, the count rates are measured in the energy windows that are associated to the peaks in the energy spectrum of the gamma photons at 32 keV, 81 keV and 356 keV.

The count rates measurements in the energy windows at 32 keV and 81 keV are mainly sensitive to the fluid fractions of fluid mixture and the constituting elements (composition) due to the photoelectric and Compton effects at these energies. The count rates measurements in the energy window at 356°keV are substantially sensitive to the density of the constituting elements due to the Compton effect only at this energy. Based on these attenuation measurements and calibration measurements, the fractional flowrate for each phase and the density of the multiphase fluid mixture 13 can be estimated. Such an estimation has been described in details in several documents, in particular WO 02/50522 and will not be further described in details herein.

The measuring apparatus 1 may also comprise a temperature sensor (not shown) for measuring the temperature of the multiphase fluid mixture 13.

In another embodiment, both sensing arrangements hereinbefore presented may be combined to estimate the total flowrate of the multiphase fluid mixture, the density of the multiphase fluid mixture and the fractional flowrate for each phase of the multiphase fluid mixture.

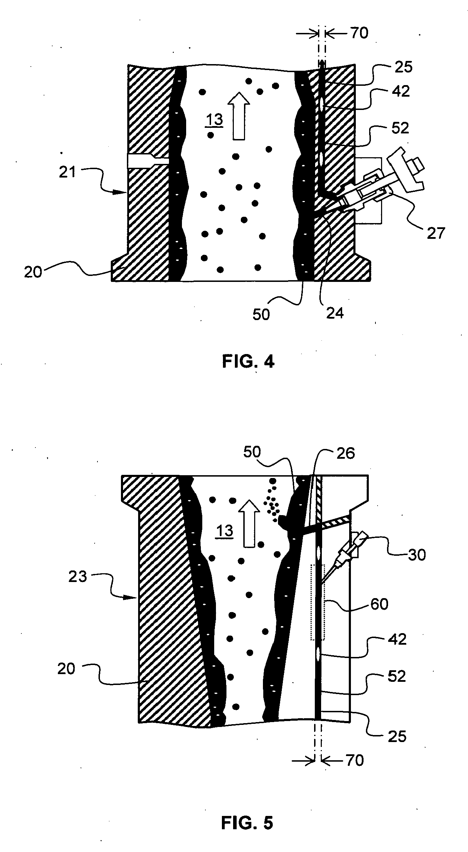

The second sensing arrangement enables measuring a second characteristic value of one of the phase of the multiphase fluid mixture flowing into the channel 25. As explained hereinafter in relation with Figures 4 and 5, the second sensing arrangement may measure the water salinity, or the water liquid ratio of a liquid

The second sensing arrangement may be an optical sensor 30. Such an optical sensor is described in details in US 5,956,132. The optical sensor 30 comprises a rod, for example made of sapphire, having a bi-cone shaped tip. The bi-cone shaped tip comprises a first zone and a second zone in contact with the multiphase fluid mixture flowing into the channel. The first zone and the second zone are adjacent and coaxial relatively to a rod longitudinal axis. The first zone forms a first angle (for example 100°) relatively to the rod longitudinal axis. Thus, an incident light beam is reflected when the tip is surrounded with gas and refracted when the tip is surrounded with liquid. The second zone forms a second angle (for example 10°) relatively to the rod longitudinal axis. Thus, it is possible to distinguish between oil and water. The reflected fraction of the incident light beam varies as a function of the refractive index of the phase of the multiphase fluid mixture in which the tip is surrounded.

The optical sensor 30 is coupled to an optical coupling device 31. The optical coupling device comprises an emitting diode, e.g. a laser diode for providing an incident light beam to the tip, and a detecting diode, e.g. a photo-transistor for detecting the reflected light beam from the tip. The detecting diode provides a signal which level is specific to the fluid phase in contact with the tip of the optical sensor. When the probe is surrounded by gas, a high level signal is detected. When the probe is surrounded by oil or gasoline, most of the incident light beam is refracted in the surrounding liquid and a low level signal is detected. When the probe is surrounded by water, an intermediate level signal is detected. As a consequence, the optical sensor 30 enables discriminating water relatively to oil and gas.

The second sensing arrangement may be an electromagnetic sensor for measuring the permittivity and conductivity of the multiphase fluid mixture flowing in the channel, in particular of each separated phase. The electromagnetic sensor can be either a coaxial probe performing reflection measurements, or a transmitter and a receiver performing transmission measurements, or an antennae performing resonance measurements, or a combination of the above. Such an electromagnetic sensor is described in details in US 6,831,470. The permittivity and conductivity of water varies with salinity and salt species. The formation water, the injection water, and the completion liquid have different salinity concentration. As a consequence, the electromagnetic sensor enables discriminating between the formation water, the injection water, and the completion liquid. Monitoring the salinity changes enables detecting a change of water composition which can be linked to the occurrence of various water breakthroughs in the produced fluid mixture.

The second sensing arrangement may be positioned closed to, before the outlet 26. Any other position of the sensor facing a zone of the channel where each separated phase to be measured has a sufficient thickness may be convenient.

It is to be noted that, though, the channel 25 and the optical detector 30 on the one hand, the pressure tappings 28, 29 and the pressure sensors on the other hand, and the gamma rays source and detector on the other hand have been depicted in the same plane, this is only for a mere drawing simplicity reason. It may be apparent for the skilled person that said entities may be positioned around the pipe section 20 in different planes.

The pressure sensors 33, 34, the temperature sensor (not shown), the gamma rays detector 36 are coupled to the control and data acquisition arrangement 16. The optical sensor 30 may be coupled to the control and data acquisition arrangement 16 through the optical coupling device 31. The valve arrangement 27 may be coupled to the control and data acquisition arrangement 16 through a valve electronic interface 32. The valve electronic interface 32 may provide the electrical power necessary for operating the valve arrangement 27.

The control and data acquisition arrangement 16 may determine the total flowrate, the flow rates of the individual phases of the multiphase fluid mixture, the density of the multiphase fluid mixture, the water liquid ratio and other values based on measurements provided by the various sensors and detectors. It may further control the operation of the valve arrangement through the valve electronic interface 32.

Figure 3 is a cross-section view in a pipe section schematically illustrating a situation of high gas volume fraction GVF. In such a situation, a main wet gas stream 40 with droplets of oil and water 51 flows in the pipe section 20, while a film of liquid comprising oil and water 50 with bubbles of gas 41 flows along the wall of the pipe section 20. At high gas volume fraction GVF the accuracy of gamma densitometer for WLR can dramatically decrease. A high gas volume fraction GVF of the multiphase fluid mixture is considered to be at least 90%. The present measuring apparatus has particular utility when the GVF is high. In particular, the sensitivity of water liquid ratio WLR measurement decreases dramatically when gas volume fraction GVF go up to 95% due to the fact that there is very little water present in the flow cross-section. For example at 95% gas volume fraction GVF and a water liquid ratio WLR of 10%, the water represents 0.5% of the total volume fraction. This affects both the water liquid ratio sensitivity and the fractions measurements, and consequently the flow rates calculation.

In order to compensate these limitations, additional measurements can be performed on the liquid phase which becomes rare. This requires sampling a multiphase fluid mixture sample from the main flow path that is enriched in the liquid phase. This liquid enriched sample is representative of the liquid mixture, which flows through the main stream of the multiphase mixture. The measuring apparatus of the invention enables collecting the liquid enriched sample and maintained this sample at the same operating conditions (pressure and temperature) than the multiphase fluid mixture in the main flow path. The measuring apparatus has the capability to adjust the residence time of the liquid sample in the channel by regulating the flow suction in order to separate the remaining bubble from the liquid. Further, the measuring apparatus has the capability to separate the oil and water phases from the liquid phase and to perform measurements related to the properties of each individual phase, for example water salinity measurement of the water phase.

Figure 4 is a cross-section view schematically illustrating an upstream part 21 of the measuring apparatus of the invention.

The combination of the pressure drop generated by the multiphase fluid mixture 13 flowing through the Venturi throat 22 from the upstream part 21 towards the downstream part 23, and of the channel 25 forming a bypass of the throat coupling the upstream part 21 to the downstream part 23 generates an aspiration effect (depicted as arrows in the liquid phase film 50) at the inlet 24 of the channel 25.

An amount of the multiphase fluid mixture enriched in liquid is sucked from the main flow path, mainly from the layer of fluid contacting the pipe section wall. A corresponding sample of the multiphase fluid mixture is diverted into and circulates through the channel 25. The aspiration effect is moderated by means of the valve arrangement 27.

As the channel 25 is positioned inside the wall thickness of the pipe section, the diverted sample of multiphase fluid mixture flows through the channel while remaining at the same temperature than the multiphase fluid mixture flowing through the Venturi throat and substantially at the same pressure than multiphase fluid mixture flowing at the downstream part.

The channel has an internal diameter 70 such that the circulation of the diverted sample of the multiphase fluid mixture is driven by a capillary effect. Thus, the diverted sample flows within the channel according to a slug flow, namely a succession of separated enriched phase samples. A succession of liquid plugs 52 separated by gas caps 42 is observed within the channel 25. As an example, the internal diameter 70 of the channel may be in the order of 1 to 4 mm.

The flow restriction or valve arrangement 27 regulates the sampled multiphase fluid mixture flow within the channel 25, by controlling the flow suction at the channel inlet and the flow speed within the channel. Width the valve arrangement 27, this may be finely controlled by opening or closing the valve. Assuming the pipe section wall at the inlet 24 is all the time wetted, an adapted suction velocity at the inlet enables catching a mixture from the liquid film with a small amount of gas (gas bubble - see Figure 3). Further, the speed of the fluid flow within the channel should be sufficiently slow in order to achieve a good stratification of the gas phase and the liquid phase within the channel, and sufficiently fast to maintain the flow direction in the small channel. Indeed, these enable controlling the residence delay of the fluid mixture flowing into the channel such that enriched liquid phase samples, or in other words separated pocket of liquid and gas easily detectable and measurable by the measuring apparatus.

Figure 5 is a cross-section view schematically illustrating a downstream part 23 of the measuring apparatus of the invention.

The succession of liquid plugs 52 separated by gas caps 42 flows within the channel 25 towards the outlet 26. The succession of gas caps and liquid plugs provide an ideal succession of easy detectable separated phases. Each liquid plug 52 forms a representative sample of the enriched liquid phase 60. The liquid plug can be detected by the second sensing arrangement 30. Said representative liquid samples are not representative in term of gas volume fraction GVF but are representative in term of liquid fractions and liquid properties. A characteristic value of said representative liquid sample 60 at free gas conditions can be measured by the second sensing arrangement 30.

For example, the second sensing arrangement 30 may measure the water salinity of the water phase, or the water liquid ratio of the liquid phase. It may also identify formation water, injection water, or completion liquid within the liquid sample.

After measurement, the diverted sample returns back to the main flow path through the outlet 26 (depicted as arrows in the liquid phase film 50).

The stratification of the diverted sample may be further improved if the measuring apparatus 1 is positioned such that the channel 25 is oriented substantially parallel to the gravity direction. In this case, both capillary effect due to the channel diameter and length, and gravity due to the channel orientation carry out the stratification of the sample which becomes a succession of gas caps and liquid plugs.

Even liquid plug comprising water and oil may be further separated in separated oil phase and water phase. Therefore, various measurements can be performed on the water sample and on the oil sample using appropriate sensors as the second sensing arrangement 30.

As depicted in Figures 4 and 5, the channel 25, the inlet 24 and the outlet 26 constitutes a sampling cell that is integrated within the pipe section 20 comprising the Venturi and enables creating multiphase fluid mixture samples locally, namely close to the zone where the samples are collected and to the zone where the properties of the samples are measured.

The measured first characteristic value (total flow rate, fractional flow rate, density, etc...) of the multiphase fluid mixture flowing into the main flow path can be corrected based on the measured second characteristic value of the liquid phase sample flowing into the channel.

As an example, if the water liquid ratio WLR measured independently with the optical sensor (second sensing arrangement) is more accurate than the one measured with the densitometer (first sensing arrangement), this value may be corrected by replacing the inaccurate value measured with the densitometer by the one measured by the optical sensor. Further, this accurate water liquid ratio WLR measurement obtained with the optical sensor can be used in the calculation performed during the densitometer processing in order to reduce the calculation to gas liquid fraction measurements. Thus, the performance of the measuring apparatus can be improved.

As another example, the electromagnetic sensor (second sensing arrangement) can be used to determine the salinity of the water fraction, and in particular to detect any change in the salinity. This is used to assess the conditions in the well bore, for example the detection of formation water, or breakthrough of injected water, or differentiation between formation water or completion fluid. In such case, the operating point of the Venturi flow meter (first sensing arrangement) can be adjusted. In effect, the densitometer needs to be calibrated by measuring the individual attenuation of various samples comprising a pure phase (gas, oil or water) which characteristics are known. When pressure and temperature change during the operation of the densitometer, the densities of phase change while their compositions stay the same. The densities variation can be tracked easily at the densitometer level. However, when the salinity of water changes, the effect on the measurements cannot be detected at the densitometer level. The variation in the salinity of water influences the attenuation of water measured by the densitometer. By measuring, the salinity of water in the multiphase fluid mixture flowing in the channel, it is possible to correct the attenuation measurements related to the multiphase fluid mixture flowing in the main path. It should be appreciated that embodiments of the present invention are not limited to onshore hydrocarbon wells and can also be used offshore. Furthermore, although some embodiments have drawings showing a horizontal well bore and a vertical well bore, said embodiments may also apply to a deviated well bore. All the embodiments of the present invention are equally applicable to cased and uncased borehole (open hole). Although particular applications of the present invention relate to the oilfield industry, other applications to other industry, for example the mining industry or the like also apply. The apparatus of the invention is applicable to various hydrocarbon exploration and production related applications, for example permanent well monitoring applications wherein several measuring apparatuses are positioned at various locations in the well.

Though, the invention is described in conjunction with a Venturi flow meter, what is important is the generation of a pressure drop when the multiphase fluid mixture flows through the flow meter. This could also be obtained with a V-cone flow meter.

The drawings and their description hereinbefore illustrate rather than limit the present invention.

Although a drawing shows different functional entities as different blocks, this by no means excludes implementations in which a single entity carries out several functions, or in which several entities carry out a single function. In this respect, the drawings are very diagrammatic.

Any reference sign in a claim should not be construed as limiting the claim. The word "comprising" does not exclude the presence of other elements than those listed in a claim. The word "a" or "an" preceding an element does not exclude the presence of a plurality of such element.

## Claims

1. An apparatus (1) for measuring at least one characteristic value of a multiphase fluid mixture (13) comprises:
- a pipe section (20) having a main flow path through which the multiphase fluid mixture (13) flows, the main flow path comprising a throat (22) between an upstream part (21) and a downstream part (23) of the main flow path such as to generate a pressure drop between the upstream part (21) and the downstream part (23);
- a first sensing arrangement (28, 33, 29, 34, 35, 36) to measure a first characteristic value of the multiphase fluid mixture (13) flowing into the main flow path;
- a channel (25) being positioned within the wall of the pipe section (20) such as to maintain similar operating conditions as in the main flow path, the channel (25) being coupled to the main flow path by an inlet (24) at the upstream part (21) and by an outlet (26) at the downstream part (23) such that the pressure drop generates an aspiration effect diverting a sample of the multiphase fluid mixture (13) flowing through the main flow path into the channel (25), the channel (25) having an internal diameter (70) such that said multiphase fluid mixture sample flows in successive separated enriched phase samples (42, 52) within the channel (25); and
- a second sensing arrangement (30) to measure a second characteristic value of one of the phase of the multiphase fluid mixture sample flowing into the channel (25).

2. The apparatus of claim 1, further comprising a valve arrangement (27) regulating a flow suction of the multiphase fluid mixture into the channel (25).

3. The apparatus of claim 2, wherein the valve arrangement (27) is positioned within the channel (25) closed to the inlet (24).

4. The apparatus according to anyone of the claims 1 to 3, wherein the apparatus is positioned such that the channel (25) is substantially parallel to the gravity direction.

5. The apparatus according to anyone of the claims 1 to 4, wherein the first sensing arrangement comprises pressure tapings (28, 29) and pressure sensors (33, 34) for measuring the differential pressure of the multiphase fluid mixture (13) between the upstream part (21) and the throat (22) and estimating a total flowrate of the multiphase fluid mixture (13).

6. The apparatus according to anyone of the claims 1 to 5, wherein the first sensing arrangement comprises a gamma rays source (35) and a gamma rays detector (36) for measuring an absorption of the gamma rays by each phase of the multiphase fluid mixture (13) and estimating a density of the multiphase fluid mixture (13) and a fractional flowrate for each phase.

7. The apparatus according to anyone of the claims 1 to 6, wherein the second sensing arrangement (30) is an optical sensor or an electromagnetic sensor.

8. The apparatus according to anyone of the claims 1 to 7, wherein the multiphase fluid mixture (13) is a hydrocarbon effluent comprising gas, oil, and water.

9. The apparatus according to anyone of the claims 1 to 8, wherein the second characteristic value relates to a liquid phase and is chosen among the group of characteristic values comprising a water salinity, water liquid ratio, formation water identification, injection water identification, completion liquid identification.

10. The apparatus according to anyone of the claims 1 to 9, wherein an inline pump is disposed within the channel (25) so as to sustain the aspiration effect.

11. A method for measuring at least one characteristic value of a multiphase fluid mixture (13) comprises:
- generating a pressure drop between an upstream part (21) and a downstream part (23) by letting flow the multiphase fluid mixture (13) in a pipe section (20) having a main flow path comprising a throat (22) positioned between the upstream part (21) and the downstream part (23) of the main flow path;
- measuring a first characteristic value of the multiphase fluid mixture (13) flowing into the main flow path by means of a first sensing arrangement;
- diverting a sample of the multiphase fluid mixture flowing through the main flow path in a channel (25) by means of an aspiration effect generated by the pressure drop, the channel (25) being positioned within the wall of the pipe section (20) such as to maintain similar operating conditions as in the main flow path, the channel (25) being coupled to the main flow path by an inlet (24) at the upstream part (21) and by an outlet (26) at the downstream part (23);
- dimensioning an internal diameter of the channel (25) such that said multiphase fluid mixture sample flows in successive separated enriched phase samples (42, 52) within the channel (25); and
- measuring a second characteristic value of one of the phase of the multiphase fluid mixture sample flowing into the channel (25) by means of a second sensing arrangement.

12. The method according to claim 11, further comprising regulating the flow in the channel (25) by means of a valve arrangement (27), the regulation comprising adjusting the residence time of the multiphase fluid mixture sample within the channel (25) such as to obtain free gas liquid samples.

13. The method according to anyone of the claims 11 to 12, further comprising positioning the channel (25) substantially parallel to the gravity direction.

14. The method according to anyone of the claims 11 to 13, further comprising correcting the measured first characteristic value of the multiphase fluid mixture (13) flowing into the main flow path based on the measured second characteristic value of one of the phase of the multiphase fluid mixture sample flowing into the channel (25).

15. The method according to anyone of the claims 11 to 14, wherein measuring the first characteristic value of the multiphase fluid mixture (13) flowing into the main flow path comprises measuring the differential pressure of the multiphase fluid mixture between the upstream and downstream parts (21, 23) and estimating a total flowrate of the multiphase fluid mixture.

16. The method according to anyone of the claims 11 to 15, wherein measuring the first characteristic value of the multiphase fluid mixture (13) flowing into the main flow path comprises submitting the multiphase fluid mixture to gamma rays, measuring an absorption of the gamma rays by each phase of the multiphase fluid mixture and estimating a fractional flowrate for each phase.

17. The method according to anyone of the claims 11 to 16, wherein measuring the second characteristic value of the liquid phase comprises measuring a water salinity, or measuring a water liquid ratio, or identifying a formation water, or identifying an injection water, or identifying a completion liquid.
